Europäisches Patentamt

European Patent Office

Office européen des brevets

(19)

(11) Numéro de publication : **0 349 428 B1**

## (12) FASCICULE DE BREVET EUROPEEN

(45) Date de publication du fascicule du brevet :
**20.01.93 Bulletin 93/03**

(51) Int. Cl.$^5$ : **A61K 9/51**

(21) Numéro de dépôt : **89401856.3**

(22) Date de dépôt : **28.06.89**

(54) **Procédé de préparation de systèmes colloidaux dispersibles d'une protéine, sous forme de nanoparticules.**

(30) Priorité : **30.06.88 FR 8808871**

(43) Date de publication de la demande :
**03.01.90 Bulletin 90/01**

(45) Mention de la délivrance du brevet :
**20.01.93 Bulletin 93/03**

(84) Etats contractants désignés :
**AT BE CH DE ES FR GB GR IT LI LU NL SE**

(56) Documents cités :
**EP-A- 0 274 961
EP-A- 0 275 796
DE-A- 1 542 261
FR-A- 1 470 723
US-A- 3 137 631
PHARM. ACTA HELV., vol. 58, no. 7, 1983,
pages 196-209, Zürich, CH; J. KREUTER:
"Evaluation of nanoparticles as drug-deliverysystems I: Preparation methods"**

(73) Titulaire : **CENTRE NATIONAL DE LA
RECHERCHE SCIENTIFIQUE (CNRS)
15, Quai Anatole France
F-75700 Paris Cedex 07 (FR)**

(72) Inventeur : **Stainmesse, Serge
8 rue Spinoza
F-94600 Choisy le Roi (FR)**
Inventeur : **Fessi, Hatem
9 rue Friant
F-75007 Paris (FR)**
Inventeur : **Devissaguet, Jean-Philippe
14 Bd d'Inkermann
F-92200 Neuilly sur Seine (FR)**
Inventeur : **Puisieux, Francis
66 rue de Strasbourg
F-94700 Maisons-Alfort (FR)**

(74) Mandataire : **Varady, Peter et al
Cabinet Lavoix 2, Place d'Estienne d'Orves
F-75441 Paris Cedex 09 (FR)**

EP 0 349 428 B1

## Description

La présente invention a pour objet un nouveau procédé de préparation de systèmes colloïdaux dispersibles d'une protéine sous forme de particules sphériques de type matriciel et de taille inférieure à 500 nm (nanoparticules).

DE-A-1542261 décrit un procédé de préparation de microcapsules dont les parois sont constitués d'un polymère, par dispersion de la matière à encapsuler dans une solution d'un polymère coagulable pour l'effet de la chaleur.

EP-A-0 275 796 a pour objet un procédé de préparation de systèmes colloïdaux dispersibles d'une substance, sous forme de particules sphériques de type matriciel et de taille inférieure à 500 nm. (nanoparticules), caractérisé en ce que :

(1) on prépare une phase liquide constituée essentiellement par une solution de la substance dans un solvant ou dans un mélange de solvants, et pouvant être additionnée d'un ou de plusieurs surfactifs,

(2) on prépare une seconde phase liquide constituée essentiellement par un non-solvant ou un mélange de non-solvants de la substance et pouvant être additionnée d'un ou de plusieurs surfactifs, le non-solvant ou le mélange de non-solvants de la substance étant miscible en toutes proportions au solvant ou mélange de solvants de la substance,

(3) on ajoute sous agitation modérée, l'une des phases liquides obtenues sous (1) ou (2) à l'autre, de manière à obtenir pratiquement instantanément une suspension colloïdale de nanoparticules de la substance, et

(4) si l'on désire, on élimine tout ou partie du solvant ou du mélange de solvants de la substance et du non-solvant ou du mélange de non-solvants de la substance, de manière à obtenir une suspension colloïdale de concentration voulue en nanoparticules ou à obtenir une poudre de nanoparticules.

La présente invention concerne une variante du procédé ci-dessus dans lequel, ladite substance est une protéine et éventuellement une substance biologiquement active, ledit solvant est l'eau ou un mélange aqueux à une température inférieure à la température de coagulation de la protéine, ledit non-solvant de la substance est l'eau à une température supérieure à la température de coagulation de la protéine et peut éventuellement contenir une substance biologiquement active, et lesdites deux phases liquides (1) et (2) sont rassemblées dans des conditions de pH éloigné du point isoélectrique de la protéine.

Ainsi, le présent procédé est caractérisé en ce que :

(1) on prépare une phase liquide constituée essentiellement par une solution de la protéine et éventuellement d'une substance biologiquement active dans l'eau ou dans une solution aqueuse à une température inférieure à la température de coagulation de la protéine, et pouvant être additionnée d'un ou de plusieurs surfactifs,

(2) on prépare une seconde phase liquide constituée essentiellement par de l'eau ou d'une solution aqueuse à une température supérieure à la température de coagulation de la protéine, pouvant contenir une substance biologiquement active et pouvant être additionnée d'un ou de plusieurs surfactifs,

(3) on ajoute sous agitation modérée, l'une des phases liquides obtenues sous (1) ou (2) à l'autre dans des conditions de pH éloigné du point isoélectrique de la protéine, de manière à obtenir pratiquement instantanément une suspension colloïdale de nanoparticules de la protéine et éventuellement de la substance biologiquement active, et

(4) si l'on désire, on élimine tout ou partie de l'eau ou du mélange aqueux, de manière à obtenir une suspension colloïdale de concentration voulue en nanoparticules ou à obtenir une poudre de nanoparticules.

La protéine est notamment une protéine naturelle telle qu'une sérumalbumine (par exemple la sérumalbumine humaine ou bovine) ou une élastine (bovine, etc). La substance biologiquement active peut être un principe actif médicamenteux ou un précurseur médicamenteux, un réactif biologique ou un principe cosmétique. L'invention permet d'obtenir des nanoparticules de protéine seule (utilisables telles quelles) ou avec la substance biologiquement active. Il est également possible et même souhaitable en cas d'instabilité thermique, de fixer la substance biologiquement active sur les nanoparticules de protéine déjà formées.

Le solvant de la phase (1) qui est l'eau ou une solution aqueuse (par exemple de l'eau acidifiée ou basifiée) se trouve notamment à une température allant de 0° à 50°C, par exemple environ à la température ambiante.

Le non-solvant de la protéine dans la phase (2), qui est de l'eau ou une solution aqueuse (par exemple de l'eau acidifiée ou basifiée) se trouve notamment à une température allant de 80° à 100°C (sous pression atmosphérique), par exemple environ à la température d'ébullition.

Le pH du mélange des phases (1) et (2) doit être éloigné du point isolélectrique de la protéine afin d'éviter sa floculation. Cette différence de pH souhaitable est de l'ordre de 2 à 3. Les protéines naturelles ayant souvent un pH de l'ordre de 5 à 6, il est souhaitable que la solution finale ait un pH d'environ 3 ou d'environ 9. A cet effet, l'acide ou la base peut être ajouté(e) indifféremment à la phase (1) ou (2).

EP 0 349 428 B1

On entend par "agitation modérée" une légère agitation de 10 à 500 rpm, p. ex. environ 100 rpm, notamment par agitateur magnétique.

La concentration de protéine dans la phase (1) peut varier de 0,1 à 10%, préférentiellement de 0,5 à 4%.

Le rapport des volumes phase (1)/phase (2) peut varier de 0,1 à 1, préférentiellement de 0,2 à 0,6.

Finalement, la solution colloïdale de nanoparticules peut être à volonté concentrée, stérilisée, tamponnée (par exemple au pH physiologique), lyophilisée ou réticulée.

L'invention permet d'obtenir des nanoparticules de protéine notamment de 150 à 300 nm.

Les exemples suivants illustrent l'invention :

Exemple 1 : Préparation de nanoparticules de sérumalbumine humaine (SAH).

```
Phase 1
SAH                                      1,0 g
Acide chlorhydrique N                    0,3 g
Eau déminéralisée ou distillée       100,0 g
à la température ambiante
Phase 2
Eau déminéralisée ou distillée portée à
l'ébullition                         180,0 g
```

La phase 1 est ajoutée sous agitation magnétique à la phase 2. Le milieu devient immédiatement opalescent par formation de nanoparticules de SAH. La taille moyenne des nanoparticules, mesurée dans un diffractomètre à rayon laser (Nanosizer [R] de chez Coultronics) est de 190 nm avec un indice moyen de dispersion de 0,5.

La suspension peut être concentrée sous pression réduite au volume désiré, par exemple 100 cm³.

Exemple 2 : Préparation de nanoparticules de sérumalbumine humaine stériles.

On procède comme dans l'exemple 1, puis la suspension est stérilisée à l'autoclave à 134°C pendant 15 minutes. La taille moyenne des particules demeure pratiquement inchangée après stérilisation.

Exemple 3 : Préparation de nanoparticules de sérumalbumine humaine lyophilisées.

On procède comme dans l'exemple 2, puis la suspension stérile est lyophilisée.

L'addition d'un cryoprotecteur (maltose, tréhalose... etc) n'est pas indispensable, mais favorise la remise en suspension du lyophilisat. La taille moyenne des particules demeure inchangée après lyophilisation.

Exemple 4 : Préparation de nanoparticules de sérumalbumine humaine réticulées.

On procède comme dans l'exemple 1, mais en ajoutant 0,06 g d'une solution aqueuse de glutaraldéhyde à 25% (p/v) à la phase 1. La taille moyenne des particules demeure inchangée après réticulation.

Exemple 5 : Préparation de nanoparticules de sérumalbumine bovine (SAB).

On procède comme dans l'exemple 1, en remplaçant la SAH par de la SAB et en remplaçant l'acide chlorhydrique normal par la même quantité de soude 0,01 N. La taille moyenne des nanoparticules est de 150 nm avec un indice moyen de dispersion de 0,5.

Les nanoparticules de SAB peuvent être réticulées, stérilisées à l'autoclave et lyophilisées comme celles de SAH.

Exemple 6 : Préparation de nanoparticules d'élastine.

On procède comme dans l'exemple 1 mais en remplaçant la SAH par de l'élastine. La taille moyenne des nanoparticules est de 280 nm avec un indice moyen de dispersion de 1.

Les nanoparticules d'élastine peuvent être réticulées, stérilisées à l'autoclave et lyophilisées comme celles de SAH.

Exemple 7 : Adsorption d'un principe actif sur des nanoparticules protéiques.

Aux nanoparticules préparées selon l'exemple 1 (SAH) ou selon l'exemple 5 (SAB) on ajoute des quantités croissantes (de 0,25g à 2,50g) de salicylate de sodium. Le taux de fixation du salicylate de sodium sur les nanoparticules, mesuré après ultracentrifugation, est de 60% de la quantité mise en oeuvre, quelle que soit la quantité de principe actif ajouté.

Exemple 8 : Préparation de nanoparticules en présence d'un principe actif.

On procède selon l'exemple 1 (1g de SAH) ou selon l'exemple 5 (1g de SAB), mais en présence de 0,50g de salicylate de sodium dissous dans la phase 1. La taille moyenne des nanoparticules est de 200 nm avec un indice moyen de dispersion de 0,5. Le taux de fixation du salicylate de sodium sur les nanoparticules, mesuré après ultracentrifugation, est de 60% de la quantité mise en oeuvre.

Exemple 9 : Variante de l'exemple 8.

On procède selon l'exemple 8, mais le salicylate de sodium est dissous dans la phase 2. Les nanoparticules obtenues ont les mêmes caractéristiques que celles de l'exemple 8.

Exemple 10 : Préparation de nanoparticules protéiques de doxorubicine.

Aux nanoparticules préparées selon l'exemple 1 (SAH) ou selon l'exemple 5 (SAB) on ajoute 50 mg de chlorhydrate de doxorubicine. Le taux de fixation de la doxorubicine sur les nanoparticules, mesuré après ultracentrifugation, est de 90% de la quantité mise en oeuvre.

## Revendications

1. Procédé de préparation de systèmes colloïdaux dispersibles d'une protéine sous forme de particules sphériques de type matriciel et de taille inférieure à 500 nm (nanoparticules), caractérisé en ce que :
   (1) on prépare une phase liquide constituée essentiellement par une solution de la protéine et éventuellement d'une substance biologiquement active dans l'eau ou dans une solution aqueuse à une température inférieure à la température de coagulation de la protéine, et pouvant être additionnée d'un ou de plusieurs surfactifs,
   (2) on prépare une seconde phase liquide constituée essentiellement par de l'eau ou d'une solution aqueuse à une température supérieure à la température de coagulation de la protéine, pouvant contenir une substance biologiquement active et pouvant être additionnée d'un ou de plusieurs surfactifs,
   (3) on ajoute sous agitation modérée, l'une des phases liquides obtenues sous (1) ou (2) à l'autre dans des conditions de pH éloigné du point isoélectrique de la protéine, de manière à obtenir pratiquement instantanément une suspension colloïdale de nanoparticules de la protéine et éventuellement de la substance biologiquement active, et
   (4) si l'on désire, on élimine tout ou partie de l'eau ou du mélange aqueux, de manière à obtenir une suspension colloïdale de concentration voulue en nanoparticules ou à obtenir une poudre de nanoparticules.

2. Procédé selon la revendication 1, caractérisé en ce que la protéine est une sérumalbumine.

3. Procédé selon la revendication 1, caractérisé en ce que la protéine est une élastine.

4. Procédé selon l'une des revendications 1 à 3, caractérisé en ce que la substance biologiquement active est un principe actif médicamenteux ou un précurseur médicamenteux, un réactif biologique ou un principe cosmétique.

5. Procédé selon l'une quelconque des revendications 1 à 3, caractérisé en ce que la substance biologiquement active est fixée sur les nanoparticules de protéine seule déjà formées dans l'étape (3).

6. Procédé selon l'une quelconque des revendications 1 à 5, caractérisé en ce que l'eau ou la solution aqueuse de la phase préparée en (1) est à une température de 0° à 50°C.

7. Procédé selon l'une quelconque des revendications 1 à 6, caractérisé en ce que l'eau ou la solution aqueuse de la phase préparée en (2) est à une température de 80° à 100°C.

8. Procédé selon l'une quelconque des revendications 1 à 7, caractérisé en ce que le pH du mélange des phases préparées en (1) et (2) est éloigné de 2 à 3 du point isoélectrique de la protéine.

9. Procédé selon l'une quelconque des revendications 1 à 8, caractérisé en ce que la concentration de protéine dans la phase préparée en (1) est de 0,1 à 10%, préférentiellement de 0,5 à 4%.

10. Procédé selon l'une quelconque des revendications 1 à 9, caractérisé en ce que le rapport des volumes phase préparée en (1)/phase préparée en (2) est de 0,1 à 1, préférentiellement de 0,1 à 0,6.

11. Procédé selon l'une quelconque des revendications 1 à 10, caractérisé en ce que dans l'étape (4) la totalité de l'eau est éliminée par lyophilisation.

12. Procédé selon l'une quelconque des revendications 1 à 11, caractérisé en ce que les nanoparticules ont une taille d'environ 150 à 300 nm.


**Patentansprüche**

1. Verfahren zur Herstellung von kolloidalen dispergierbaren Proteinsystemen, in der Form von sphärischen Partikeln vom Typ einer Matrize und mit einer Größe, die kleiner als 500 nm (Nanopartikel) ist, dadurch gekennzeichnet, daß

(1) eine flüssige Phase bereitet wird, die im wesentlichen aus einer Lösung des Proteins und eventuell einer Substanz besteht, die in Wasser oder in einer wäßrigen Lösung bei einer Temperatur biologisch aktiv ist, die niedriger als die Koagulationstemperatur des Proteins ist, und zu der ein oder mehrere grenzflächenaktive Stoffe zugefügt werden können,

(2) eine zweite flüssige Phase bereitet wird, die im wesentlichen aus Wasser oder einer wäßrigen Lösung besteht bei einer Temperatur, die höher als die Koagulationstemperatur des Proteins liegt, und eine Substanz enthalten kann, die biologisch aktiv ist, und zu der ein oder mehrere grenzflächenaktive Stoffe zugefügt werden können,

(3) unter mäßigem Rühren eine der unter (1) oder (2) erreichten flüssigen Phasen zu der anderen bei einem pH-Wert hinzuzufügen, der vom isoelektrischen Punkt des Proteins entfernt ist, um praktisch unmittelbar eine kolloidale Suspension von Protein-Nanopartikeln und eventuell der biologisch aktiven Substanz zu bekommen, und

(4) falls gewünscht, das gesamte oder einen Teil des Wassers oder der wäßrigen Lösung eliminiert wird, um eine kolloidale Suspension gewünschter Konzentration von Nanopartikeln zu bekommen oder um ein Pulver von Nanopartikeln zu bekommen.

2. Verfahren nach Auspruch 1, dadurch gekennzeichnet, daß das Protein ein Serumalbumin ist.

3. Verfahren nach Anspruch 1, dadurch gekennzeichnet, daß das Protein ein Elastin ist.

4. Verfahren nach einem der Ansprüche 1-3, dadurch gekennzeichnet, daß die biologisch aktive Substanz ein medizinisch aktiver Wirkstoff oder ein medikamentöser Zwischenstoff, ein biologisches Reagens oder ein kosmetischer Wirkstoff ist.

5. Verfahren nach einem der Ansprüche 1-3, dadurch gekennzeichnet, daß die biologisch aktive Substanz auf Nanopartikeln von einzelnen Proteinen fixiert ist, die während des Schrittes (3) schon gebildet werden.

6. Verfahren nach einem der Ansprüche 1-5, dadurch gekennzeichnet, daß das Wasser oder die wäßrige Lösung der in (1) bereiteten Phase sich auf einer Temperatur von 0-50°C befindet.

7. Verfahren nach einem der Ansprüche 1-6, dadurch gekennzeichnet, daß das Wasser oder die wäßrige Lösung der in (2) bereiteten Phase sich auf einer Temperatur von 80-100°C befindet.

8. Verfahren nach einem der Ansprüche 1-7, dadurch gekennzeichnet, daß der pH-Wert der Mischung der in (1) und (2) bereiteten Phasen 2 bis 3 vom isoelektrischen Punkt des Proteins entfernt ist.

9. Verfahren nach einem der Ansprüche, 1-8, dadurch gekennzeichnet, daß die Konzentration des Proteins in der in (1) bereiteten Phase 0,1-10%, vorzugsweise 0,5-4% beträgt.

10. Verfahren nach einem der Ansprüche 1-9, dadurch gekennzeichnet, daß das Volumenverhältnis Phase bereitet in (1)/Phase bereitet in (2) 0,1-1, vorzugweise 0,1-0,6 beträgt.

11. Verfahren nach einem der Ansprüche 1-10, dadurch gekennzeichnet, daß in Schritt (4) das gesamte Wasser durch Lyophilisation eliminiert wird.

12. Verfahren nach einem der Anspruche 1-11, dadurch gekennzeichnet, daß die Nanopartikel eine Größe von etwa 150-300 nm aufweisen.

## Claims

1. Process for the preparation of dispersible colloidal systems of a protein in the form of spherical particles of the matrix type and less than 500 nm in size (nanoparticles), characterised in that:

   (1) a liquid phase consisting essentially of a solution of the protein and optionally of a biologically active substance in water or in an aqueous solution at a temperature below the coagulation temperature of the protein, and to which may be added one or more surfactants, is prepared,

   (2) a second liquid phase consisting essentially of water or of an aqueous solution at a temperature above the coagulation temperature of the protein, which may contain a biologically active substance and to which may be added one or more surfactants, is prepared,

   (3) one of the liquid phases obtained in (1) or (2) is added with moderate stirring to the other under conditions of pH far removed from the isoelectric point of the protein so as to obtain almost instantaneously a colloidal suspension of nanoparticles of the protein and, where appropriate, of the biologically active substance, and

   (4) if desired all or part of the water or aqueous mixture is removed so as to obtain a colloidal suspension of nanoparticles of the desired concentration or to obtain a powder of nanoparticles.

2. Process according to Claim 1, characterised in that the protein is a serum albumin.

3. Process according to Claim 1, characterised in that the protein is an elastin.

4. Process according to one of Claims 1 to 3, characterised in that the biologically active substance is a medicinal active principle or a medicinal precursor, a biological reagent or a cosmetic principle.

5. Process according to any one of Claims 1 to 3, characterised in that the biologically active substance is bound to the nanoparticles of the protein alone which are already formed in step (3).

6. Process according to any one of Claims 1 to 5, characterised in that the water or aqueous solution of the phase prepared in (1) is at a temperature of 0° to 50°C.

7. Process according to any one of Claims 1 to 6, characterised in that the water or aqueous solution of the phase prepared in (2) is at a temperature of 80° to 100°C.

8. Process according to any one of Claims 1 to 7, characterised in that the pH of the mixture of the phases prepared in (1) and (2) differs by 2 to 3 units from the isoelectric point of the protein.

9. Process according to any one of Claims 1 to 8, characterised in that the protein concentration in the phase prepared in (1) is from 0.1 to 10%, and preferably from 0.5 to 4%.

10. Process according to any one of Claims 1 to 9, characterised in that the ratio of the volumes phase prepared in (1)/phase prepared in (2) is from 0.1 to 1, and preferably from 0.1 to 0.6.

11. Process according to any one of Claims 1 to 10, characterised in that, in step (4), all of the water is removed by lyophilisation.

12. Process according to any one of Claims 1 to 11, characterised in that the nanoparticles are about 150 to 300 nm in size.